# EUROPEAN PATENT SPECIFICATION

(11) **EP 1 612 218 B1**
(45) Date of publication and mention of the grant of the patent: **14.05.2008**
(21) Application number: 04015231.6
(22) Date of filing: 29.06.2004
(51) Int. Cl.: C07K 14/22, C12N 15/31

(54) **Surface protein of Neisseria bacteria**
Oberflächenprotein aus Neisserien
Protéine de surface du Neisseria

(43) Date of publication of application: 04.01.2006
(73) Proprietor: Center for Disease Control Department of Health Taiwan, Taipei, Taiwan 100 R.O.C. (TW)
(72) Inventor: Yang, Chiou-Ying, 402 Taichung (TW)
(74) Representative: Beck, Michael Rudolf

(56) References cited:
- WO-A-00/44890
- WO-A-99/57280
- TETTELIN H ET AL: "COMPLETE GENOME SEQUENCE OF NEISSERIA MENINGITIDIS SEROGROUP B STRAIN MC58" SCIENCE, AMERICAN ASSOCIATION FOR THE ADVANCEMENT OF SCIENCE,, US, vol. 287, 2000, pages 1809-1815, XP000914963 ISSN: 0036-8075 -& DATABASE UniProt 1 October 2000 (2000-10-01), "Hypothetical protein NMB1468" XP002321105 retrieved from EBI Database accession no. Q9JYR2

### SEQUENCE LISTING

<110> Center for Disease Control Department of Health Taiwan
<120> Surface protein of Neisseria bacterium
<130> PEPLR01
<160> 10
<170> Patent In version 3.1
<210> 1 <211> 342 <212> DNA <213> Neisseria meningitidis
<400> 1
<210> 2 <211> 114 <212> PRT <213> Neisseria meningitidis
<400> 2
   Met Lys Lys Leu Leu Ile Ala Ala Met Met Ala Ala Ala Leu Ala Ala
   1                   5                        10                       15
   Cys Ser Gln Glu Ala Lys Gln Glu Val Lys Glu Ala Val Gln Ala Val
                   20                       25                       30
   Glu Ser Asp Val Lys Asp Thr Ala Ala Ser Ala Ala Glu Ser Ala Ala
               35                       40                       45
   Ser Ala Val Glu Glu Ala Lys Asp Gln Val Lys Asp Ala Ala Ala Asp
           50                       55                       60
   Ala Lys Ala Ser Ala Glu Glu Ala Val Thr Glu Ala Lys Glu Ala Val
   65                       70                       75                       80
   Thr Glu Ala Lys Glu Ala Val Thr Glu Ala Ala Lys Asp Thr Leu Asn
                       85                       90                       95
   Lys Ala Ala Asp Ala Thr Gln Glu Ala Ala Asp Lys Met Lys Asp Ala
                   100                      105                      110
   Ala Lys
<210> 3
   <211> 321
   <212> DNA
   <213> Neisseria meningitidis
<400> 3
<210> 4
   <211> 107
   <212> PRT
   <213> Neisseria meningitidis
<400> 4
   Met Lys Lys Leu Leu Ile Ala Ala Met Met Ala Ala Ala Leu Ala Ala
   1 5 10 15
   Cys Ser Gln Glu Ala Lys Gln Glu Val Lys Glu Ala Val Gln Ala Val
   20 25 30
   Glu Ser Asp Val Lys Asp Thr Ala Ala Ser Ala Ala Glu Ser Ala Ala
   35 40 45
   Ser Ala Val Glu Glu Ala Lys Asp Gln Val Lys Asp Ala Ala Ala Asp
   50 55 60
   Ala Lys Ala Ser Ala Glu Glu Ala Val Thr Glu Ala Lys Glu Ala Val
   65 70 75 80
   Thr Glu Ala Ala Lys Asp Thr Leu Asn Lys Ala Ala Asp Ala Thr Gln
   85 90 95
   Glu Ala Ala Asp Lys Met Lys Asp Ala Ala Lys
   100 105
<210> 5
   <211> 363
   <212> DNA
   <213> Neisseria meningitidis
<400> 5
<210> 6
   <211> 121
   <212> PRT
   <213> Neisseria meningitidis
<400> 6
   Met Lys Lys Leu Leu Ile Ala Ala Met Met Ala Ala Ala Leu Ala Ala
   1 5 10 15
   Cys Ser Gln Glu Ala Lys Gln Glu Val Lys Glu Ala Val Gln Ala Val
   20 25 30
   Glu Ser Asp Val Lys Asp Thr Ala Ala Ser Ala Ala Glu Ser Ala Ala
   35 40 45
   Ser Ala Val Glu Glu Ala Lys Asp Gln Val Lys Asp Ala Ala Ala Asp
   50 55 60
   Ala Lys Ala Ser Ala Glu Glu Ala Val Thr Glu Ala Lys Glu Ala Val
   65 70 75 80
   Thr Glu Ala Lys Glu Ala Val Thr Glu Ala Lys Glu Ala Val Thr Glu
   85 90 95
   Ala Ala Lys Asp Thr Leu Asn Lys Ala Ala Asp Ala Thr Gln Glu Ala
   100 105 110
   Ala Asp Lys Met Lys Asp Ala Ala Lys
   115 120
<210> 7
   <211> 384
   <212> DNA
   <213> Neisseria meningitidis
<400> 7
<210> 8
   <211> 128
   <212> PRT
   <213> Neisseria meningitidis
<400> 8
   Met Lys Lys Leu Leu Ile Ala Ala Met Met Ala Ala Ala Leu Ala Ala
   1 5 10 15
   Cys Ser Gln Glu Ala Lys Gln Glu Val Lys Glu Ala Val Gln Ala Val
   20 25 30
   Glu Ser Asp Val Lys Asp Thr Ala Ala Ser Ala Ala Glu Ser Ala Ala
   35 40 45
   Ser Ala Val Glu Glu Ala Lys Asp Gln Val Lys Asp Ala Ala Ala Asp
   50 55 60
   Ala Lys Ala Ser Ala Glu Glu Ala Val Thr Glu Ala Lys Glu Ala Val
   65 70 75 80
   Thr Glu Ala Lys Glu Ala Val Thr Glu Ala Lys Glu Ala Val Thr Glu
   85 90 95
   Ala Lys Glu Ala Val Thr Glu Ala Ala Lys Asp Thr Leu Asn Lys Ala
   100 105 110
   Ala Asp Ala Thr Gln Glu Ala Ala Asp Lys Met Lys Asp Ala Ala Lys
   115 120 125
<210> 9
   <211> 342
   <212> DNA
   <213> Neisseria gonorrhoeae
<400> 9
<210> 10
   <211> 114
   <212> PRT
   <213> Neisseria gonorrhoeae
<400> 10
   Met Lys Lys Leu Leu Ile Ala Ala Met Met Ala Ala Ala Leu Ala Ala
   1 5 10 15
   Cys Ser Gln Glu Ala Lys Gln Glu Val Lys Glu Ala Ala Gln Ala Val
   20 25 30
   Glu Ser Asp Val Lys Asp Thr Ala Ala Ser Ala Ala Glu Ser Ala Ala
   35 40 45
   Ser Ala Val Glu Glu Ala Lys Gly Gln Val Lys Asp Ala Ala Ala Asp
   50 55 60
   Ala Lys Ala Ser Ala Glu Glu Ala Val Thr Glu Ala Lys Asp Ala Ala
   65 70 75 80
   Ala Glu Thr Lys Glu Ala Val Ser Glu Ala Ala Lys Asp Thr Leu Asn
   85 90 95
   Lys Ala Ala Asp Ala Ala Gln Glu Ala Ala Asp Lys Met Lys Asp Ala
   100 105 110
   Ala Lys

## Description

### Field of the invention

The present invention discloses a conserved surface protein of Neisseria bacteria, nucleotide sequences encoding such polypeptides, and its monoclonal antibody which can be made into pharmaceutical compositions including prophylactic, diagnostic or therapeutic compositions.

### Background of the Invention

Neisseria meningitidis, a capsulated, Gram-negative bacterium, is a cause of life-threatening invasive bacterial infection, especially in young children. The organism can be classified into at least 13 serogroups based on chemically and antigenically distinctive polysaccharide capsules. Among them, serogroups A, B, C, W-135, and Y account for virtually all pathogenic isolates. Despite several decades of research, no effective vaccine that protects against all meningococcal strains is available, and diseases caused by N. meningitidis, meningitis and septicemia, remain a serious public health problem throughout the world (Peltola, Drugs 55: 347-366, 1998). The failure in development of effective vaccines is possibly attributed to the high antigenic diversity of the pathogen. The currently licensed vaccine is polysaccharide-based which does not include the serogroup B capsular polysaccharide, due to the poor immunogenicity of the latter substance (Frasch,. Meningococcal vaccines: past, present and future. In: Meningococcal Disease. Cartwright K. (Ed.) Wiley Press, New York, USA: 145-157, 1995.). Therefore effective vaccine against disease caused by serogroup B strains, the organisms responsible for the majority of meningococcal infections in many countries, is still not available (Verheul et al., Microbiol Rev 57:34-49, 1993).

For development of effective vaccines against serogroup B meningococci, most research has focused on the outer membrane proteins (OMPs). Of the five major OMP classes, class I OMP (also named PorA) has attracted the most attentions. This is because that PorA is expressed by most meningococci and is highly immunogenic in humans following infection or immunization (Guttormen et al., Infect. Immun 62:1437, 1994; Clasassen et al., Vaccine 14:1001, 1996; van der Ley and Poolman, Infect Immun. 60:3156, 1992). Moreover, specific antibodies induced by PorA exhibit both bactericidal activity and opsonic function (Aase et al., Scand J Immunol 47:388-396, 1998;Lehmann et al., Infect. Immun. 67:2552, 1999). However, the high degrees of antigenic and phase variation of PorA have limited the effectiveness of PorA-based vaccines to the vaccine-type strains (Fischer et al., Vaccine 17: 2377-2383, 1999). Broadened protection may be achieved by combining different PorA subtypes. For example, a hexavalent PorA vaccine composed of the most prevalent PorA variants found in the Netherlands and Western Europe has been developed (Claassen et al., 1996, Vaccine 14:1001-1008; Peeters et al, 1996, Vaccine 14:1009-1015). Nevertheless, the observation that different PorA phenotypes can emerge rapidly after its epidemic spread (Jelfs et al., Clin. Diagn. Lab. Immunol. 7:390-395, 2000; Martin et al., Vaccine 18:2476-2481, 2000) has to be considered in the development of PorA-based vaccine.

To create vaccines with a broad spectrum of protection, it is important to identify surface proteins which are highly conserved in different strains. Several proteins meet the criteria have been reported. These include P64k (USPTO No. 5286484), hemoglobin receptor (USPTO No. 6121037), NspA (USPTO No. 6287574 B1), NhhA (USPTO No. 6607729 B2), NMASP (USPTO No. 6693186 B2), NadA (Comanducci et al., J. Exp. Med. 195:1445-1454, 2002), GNA1870 (Masignani et al., J. Exp. Med. 197:789-799, 2003), GNA33, GNA992, GNA 1162, GNA1220, GNA1946, GNA2001 and GNA2132, in which GNA1870, GNA33, GNA1162, GNA1946 and GNA2132 are lipoproteins identified by whole genome sequencing (Pizza et al., Science 287:1816-1820, 2000). Despite the discovery of these proteins, it is still desirable to isolate more surface proteins of N. meningitidis which in combination with others may enhance the vaccine efficacy.

SEQ ID NO. 3 and 4 are known from WO 9957280 A2 and WO 0044890 A1.

### Brief summary of the invention

The present invention is related to a monoclonal antibody 4-7-3, prepared from mouse immunized with the whole cells of N. meningitidis, and its target antigen (referred to hereafter as Ag473). The antigen, estimated 10-15 kDa, is a novel lipoprotein with unknown function ubiquitous on the surface of Neisseria bacteria, including among others N. meningitidis and N. gonorrhoeae.

The present invention is related to two variants of Ag473 with SEQ ID NO. 5 as the isolated polynucleotide and SEQ ID NO.6 as the isolated lipoprotein (as shown in Sequence List), wherein SEQ ID NO. 5 and 6 are acquired from N. meningitidis.

The invention provides an isolated lipoprotein according to claim 1 and an isolated polynucleotide according to claim 5. Advantageous embodiments are laid down in further claims.

The present invention further encompasses the Ag473 in recombinant form and the antiserum raised using this protein. The antiserum can bind to living N. meningitidis resulting in bactericidal activity, showing that Ag473 has the potential to be a vaccine component and a therapeutic target.

The present invention also examines the occurrence of Ag473 protein and the corresponding gene in a number of bacterial species, using 4-7-3 antibody and colony-PCR, respectively. Positive results are only observed in the Neisseria bacteria, including N. meningitidis and N. gonorrhoeae. Therefore, the present invention also provides a novel way to diagnose Neisseria bacteria.

### Brief description of the drawings

The present invention will be better understood from the following detailed description of preferred embodiments of the invention, taken in conjunction with the accompanying drawings, in which
- FIG.1: shows a Western blot of the total proteins from cells of serogroups A, B, C, W-135, and Y N. meningitidis in which monoclonal antibody 4-7-3 was used as the primary antibody.
- FIG.2: shows the silver-stained 2D-gel (A) and its corresponding Western blot (B) of the total protein from N. meningitidis identifying the antigen for antibody 4-7-3.
- FIG.3: shows the Western blots of the total proteins from N. meningitidis (lane 3), E. coli expressing the recombinant Ag473 (lane 2) and N. meningitidis in which the corresponding gene is disrupted (lane 1), wherein (A) and (B) were probed with 4-7-3 and anti-PorA, respectively.
- FIG.4: shows a photograph of ethidium bromide-stained polyacrylamide gel of the PCR products (A) amplified with the primers flanking the 21-bp repeat region of the four gene variants as depicted on panel B. The regions where primers annealed with are underlined and marked with arrows in FIG. 7.
- FIG.5: shows the binding of anti-Ag473 antiserum to intact N. meningitidis determined by ELISA (5A) and the fluorescent activated cell scanning (FACS) analysis (5B).
- FIG.6: aligns SEQ ID No. 9 (query) with the homologue from the genome of N. gonorrhoeae (sbjct).
- FIG.7: aligns SEQ ID No. 1 (Ag473-2) with SEQ ID NO. 9 (Ag473-Ng), wherein the mismatched nucleotides are capitalized and the 21-bp repeat are italicized.
- FIG.8: aligns the amino acid sequences of the five Ag473 variants (SEQ ID No 2, 4, 6, 8, and 10); wherein Ag473-1 to Ag473-4 are sequences acquired from N. meningitidis and Ag473-Ng from N. gonorrhoeae.
- FIG.9: shows a Western blot of the total proteins from N. meningitidis (Nm) and N. gonorrhoeae (Ng) detected by monoclonal antibody 4-7-3.

The Sequence List shows the nucleotide and amino acid sequences of the five Ag473 variants described in the present invention: Ag473-2, Accession No. AY566588 (seq. no. 1); Ag473-1, Accession No. AY566589 (seq. no. 3); Ag473-3, Accession No. AY566590 (seq. no. 5); Ag473-4, Accession No. AY566591 (seq. no. 7); Ag473-Ng, Accession No. AY566592 (seq. no. 9).

### Description of the preferred embodiment

The following descriptions of the preferred embodiments are provided for the purpose to understand the features and the structures of the present invention.

The present invention uses the whole cells of serogroup B strain Nm22209 isolated in Taiwan as the antigen to prepare antibodies which can be used as a tool for finding surface components of N. meningitidis with potential to become a vaccine composition. To keep the completeness of the whole cell, bacteria were heated and injected directly for immunization.

### 1. Preparation and characterization of monoclonal antibody (mAb) 4-7-3

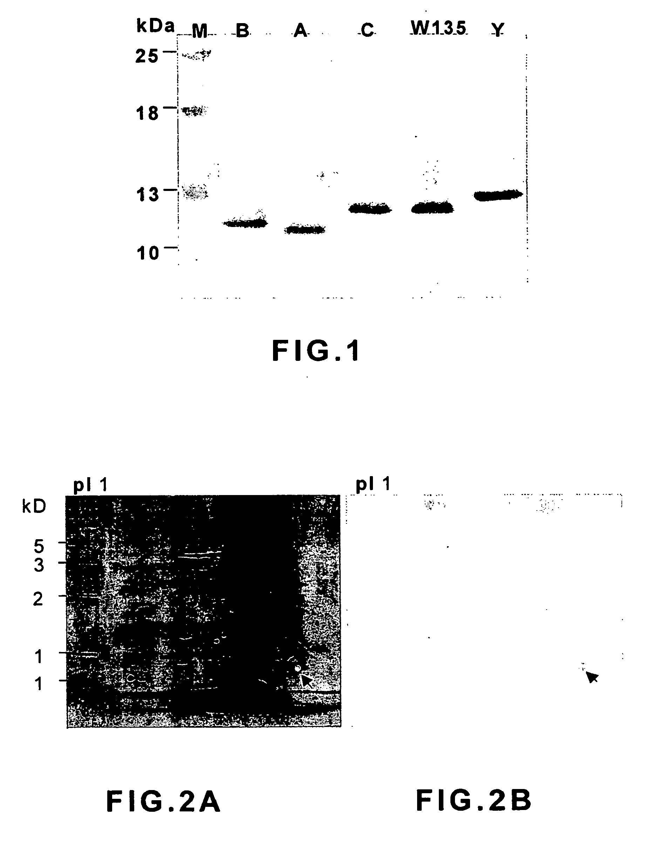

(A) Mice are injected intraperitoneally with the heat-killed Nm22209 cells.
(B) Hybridomas are obtained by the fusion of spleen cells of the immunized mouse with Sp2/0-Ag14 cells.
(C) Hybridomas secreting antibodies against Nm22209 are identified by ELISA using the supernatant harvested from a culture of hybridoma cells.
(D) Supernatant harvested from hybridoma 4-7-3 shows binding activity to all N. meningitidis tested including 184 strains isolated in Taiwan and 3 ATCC reference strains (ATCC 13077, ATCC 13090, and ATCC 13102 representing serogroups A, B, and C, respectively) but not the human neuroblastoma cell line IMR-32.
(E) The bactericidal activity of purified 4-7-3 against Nm22209 is tested according to the procedures described by Martin et. al. (J. Exp. Med. 185: 1173-1183, 1997) with the following modifications. Eighty µl of the bacterial suspension (I x 105 CFU/ml) is incubated with 10 µl of different concentrations of protein LA-purified 4-7-3 for 20 min at 37°C,[then] 10 µl of human complement (40 CH50 unit/ml, Sigma Cat# S1764) is then added to the mixture. After incubation for 1 hr, 10 µl of each mixture is plated onto chocolate agar plates. The surviving colonies are counted after 18 hr of incubation of the plates in an atmosphere of 5% CO₂ at 37°C. It is found that approximately 6 µg of 4-7-3 is required to render a 50% inhibition of bacterial growth in the experimental conditions.
(F) To reveal the entity of the antigen recognized by 4-7-3, meningococci of the five different serogroups are lysed with the SDS-PAGE loading buffer (0.025 M Tris pH 8.7, 2% SDS, 10% glycerol, 0.05 mg/ml bromophenol blue), and subjected to Western blot analysis, wherein a distinct immunoreactive band located between 10-15 kDa, which may show a slight variation in size among strains, is observed from each isolate (Fig. 1).

The above results clearly demonstrate that 4-7-3 recognizes a conserved surface protein, which is able to induce a protective immune response against N. meningitidis.

### 2. Identification of the antigen for 4-7-3 (referred to as Ag473 hereafter)

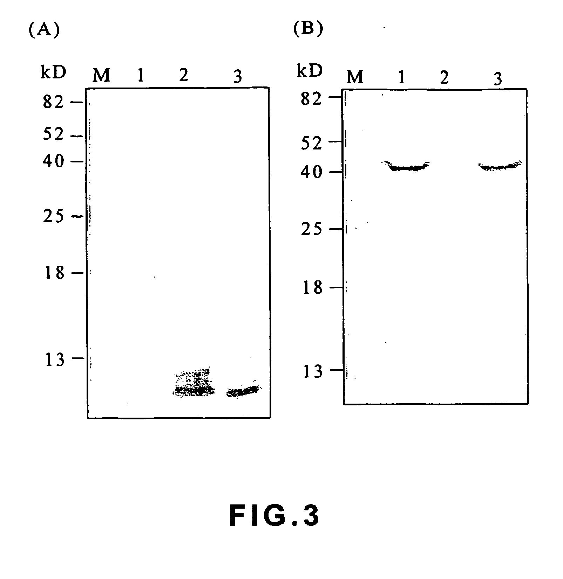

(A) Total proteins of the Nm22209 are separated by 2D gel electrophoresis in duplicate.
(B) One of the gels is subjected to silver staining and the other is used for blotting followed by probing with 4-7-3 to localize the Ag473 (as shown in FIG.2).
(C) The gel containing the silver-stained spot corresponding to the immunospot in the Western blot is collected and analyzed by mass spectrometry, which shows that it can be a hypothetical protein NMB1468 composed of 107 amino acid residues in serogroup B meningococcal strain MC58. DOLOP analysis (Madan Babu and Sankaran, Bioinformatics 18:641-643, 2002) shows that this hypothetical protein can be a lipoprotein. Though the DNA sequence is also present in the genome of serogroup A meningococcal strain A Z2491 (AL162756), it is included as a part of protein annotated to encode a 979 aa.
(D) Related art shows that the promoter of NM can function in E. coli. (Sawaya et al., Gene 233:49-57, 1999). To clarify if NMB1468 is the homolog of Ag473, PCR primers are designed according to the genome sequence of MC58. The DNA fragment encompassing the annotated open reading frame and the upstream 329 bp is amplified from Nm22209 by PCR, cloned into vector pGEM-T-Easy, and transformed into E. coli HB101. Total proteins from the transformed and non-transformed HB101 are subjected to Western blot analysis. In the meantime, a genetic engineered Nm22209 with a disrupted Ag473 gene is also generated. Therein, the recombinant protein expressed in transformed E. coli can be identified by antibody 4-7-3, but no immunoreactive bands are observed in the mutated Nm22209 (as shown in FIG.3).

The above results clearly demonstrate that Ag473 is a homolog of NMB1468 which is an expressed protein in N. meningitids.

### 3. Sequence analysis of the Ag473 gene

Sequence analysis of the above PCR product reveals (SEQ ID NO.1) an additional 21-bp direct repeat in the coding region comparing to that (SEQ ID NO.3) of NMB1468 in MC58. This repeat is not found in neither the genome sequence of MC58 nor Z2491 strains. As a result, Ag473 comprises 114 amino acid residues with a two 7-amino acid (Glu-Ala-Val-Thr-Glu-Ala-Lys) tandem repeats (SEQ ID NO.2; those underlined).

Western blotting shows that the immunoreactive bands detected by 4-7-3 are slightly varied in size among different meningococcal strains (as shown in FIG.1), which implies that there might be different variants of Ag473.

To investigate the degrees of variation, the Ag473 genes of ten meningococcal strains, including the above five strains used in the Western blot analysis, are amplified by PCR, cloned into plasmid pGEM-T-Easy, and subjected to nucleotide sequencing. Four sequences (SEQ ID NO.1, 3, 5, 7) different mainly on the numbers of the 21-bp direct repeat (those underlined) are found. The smallest gene has the same sequence (SEQ ID NO.3) as NMB1468 with only one 21-bp repeat while the longest gene has four 21-bp repeats in tandem (SEQ ID NO.7). As a consequence, the coding proteins contain 107, 114, 121 and 128 amino acids, respectively. (Please refer to SEQ ID NO.2, 4, 6, 8 and FIG.8)

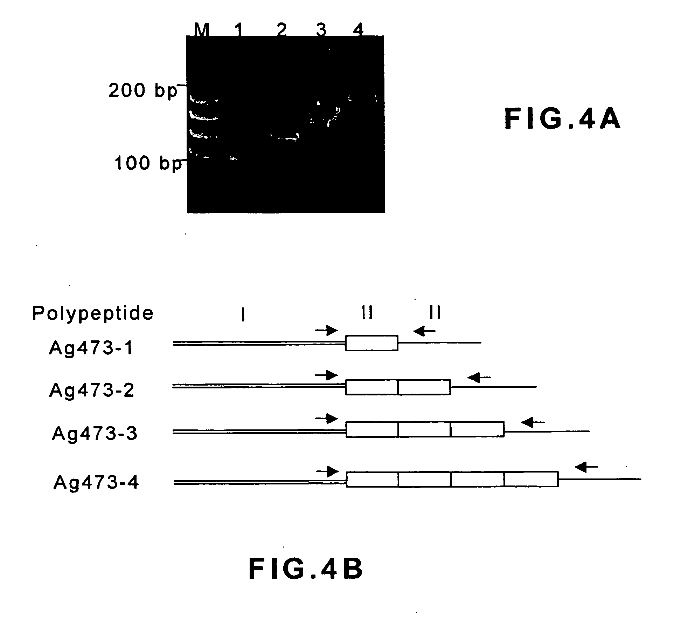

To access the numbers of Ag473 gene allele in meningococcal population, DNA fragments spanning the 21-bp repeat region are obtained from 141 strains, including the three ATCC strains mentioned above, by PCR amplification with the primers flanking the repeated region and the products are analyzed by polyacrylamide gel electrophoresis which shows that only four different sizes are present. A representative result is shown in FIG. 4A. The regions that the primers annealed to are indicated with an arrow in the Ag473-2 sequence of FIG.7 and SEQ ID NO.1.

### 4. The immunogenicity test of the recombinant Ag473 (rAg473):

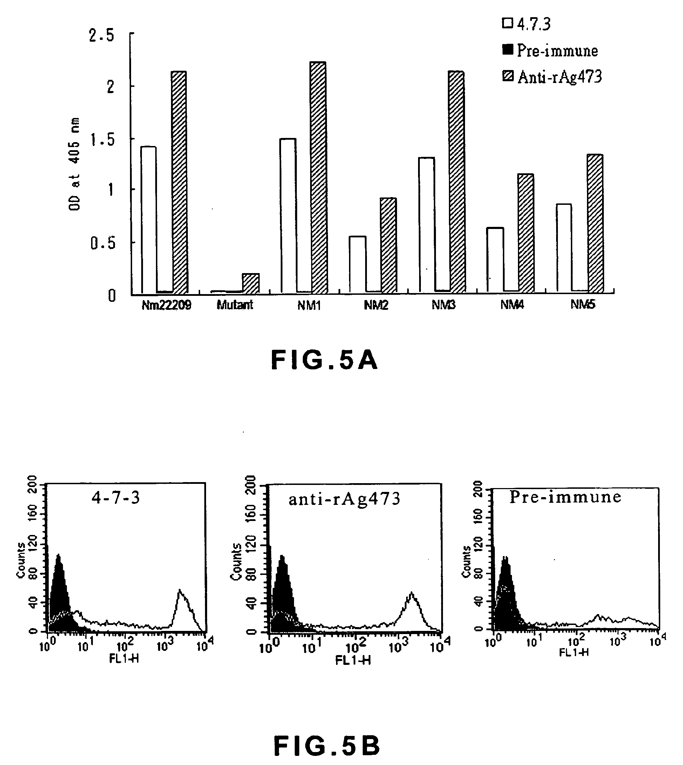

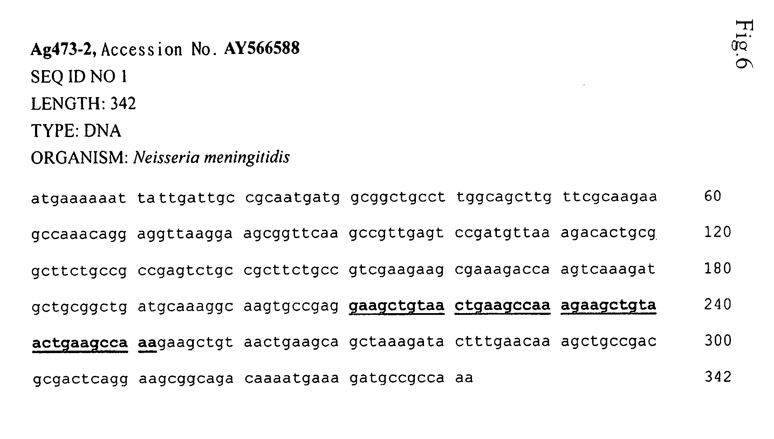

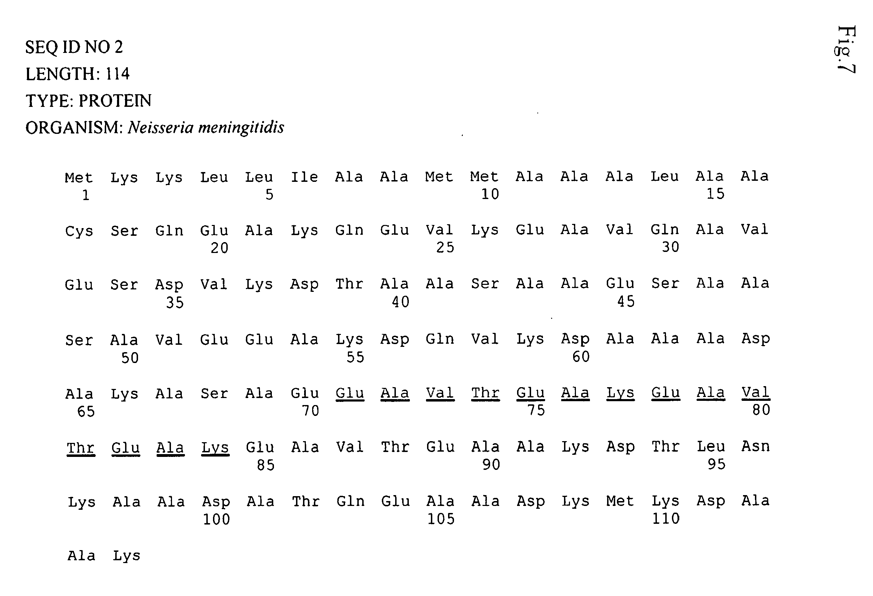

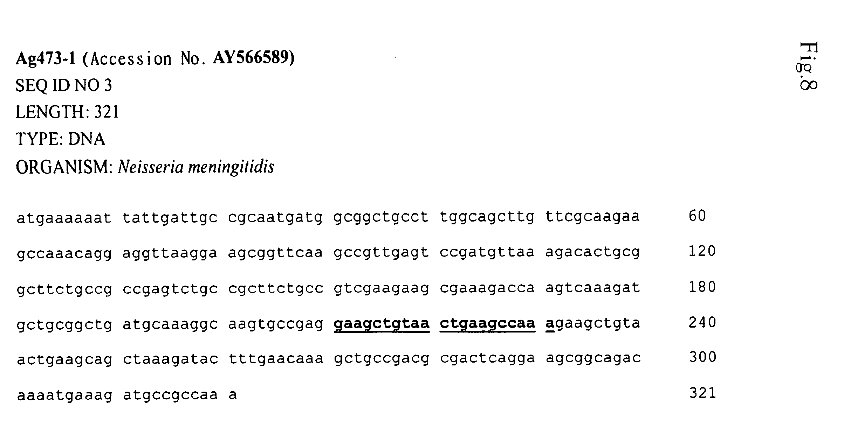

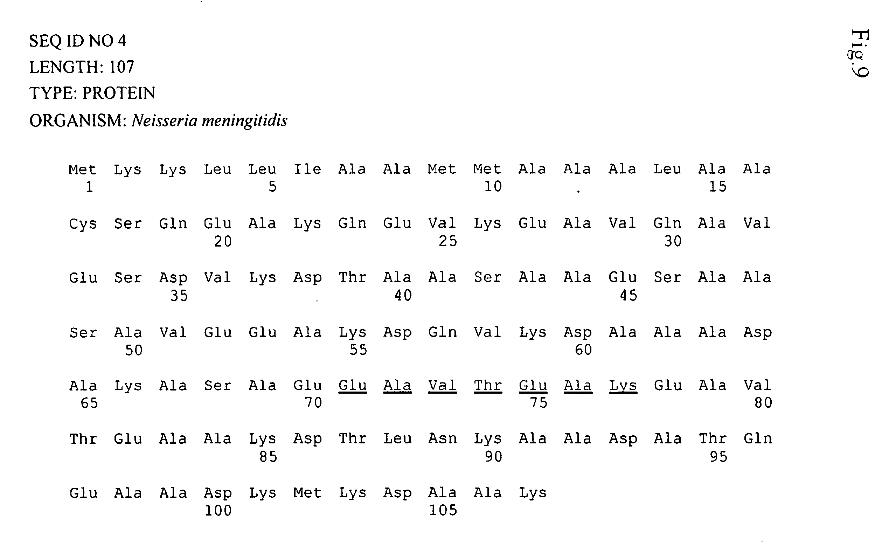

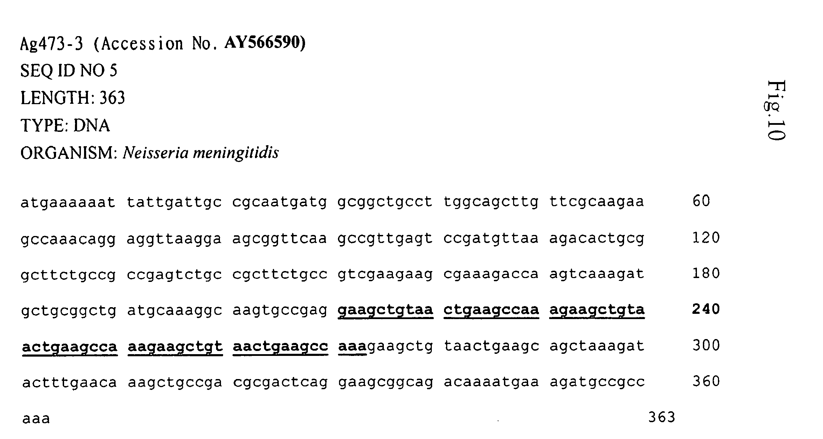

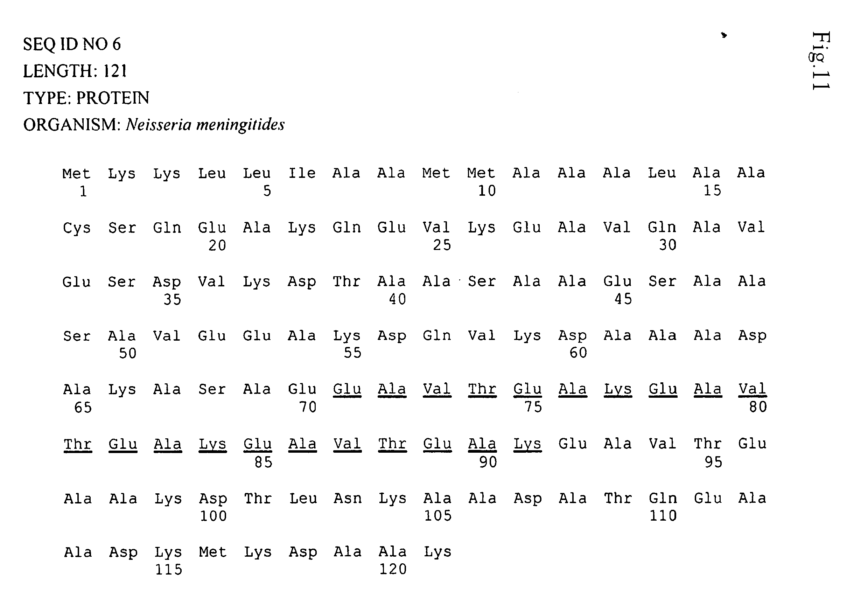

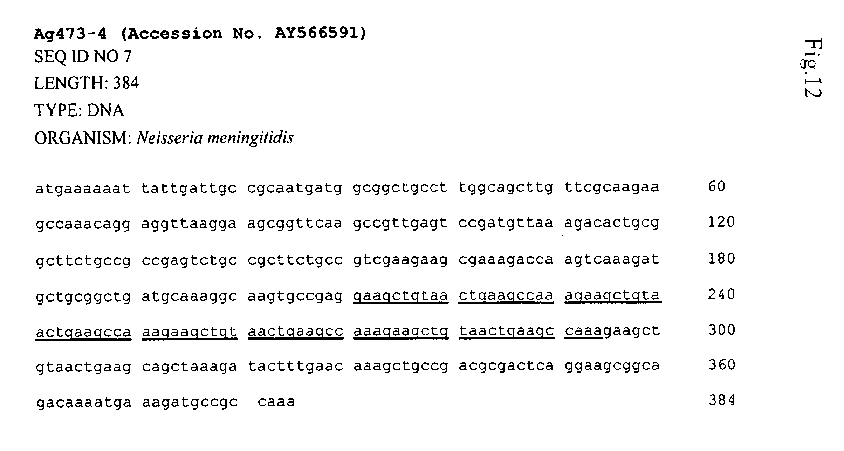

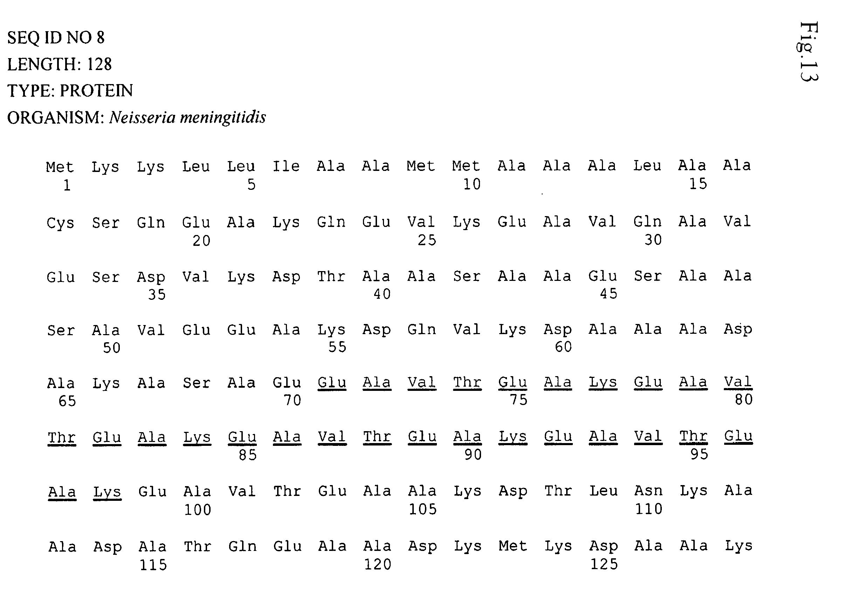

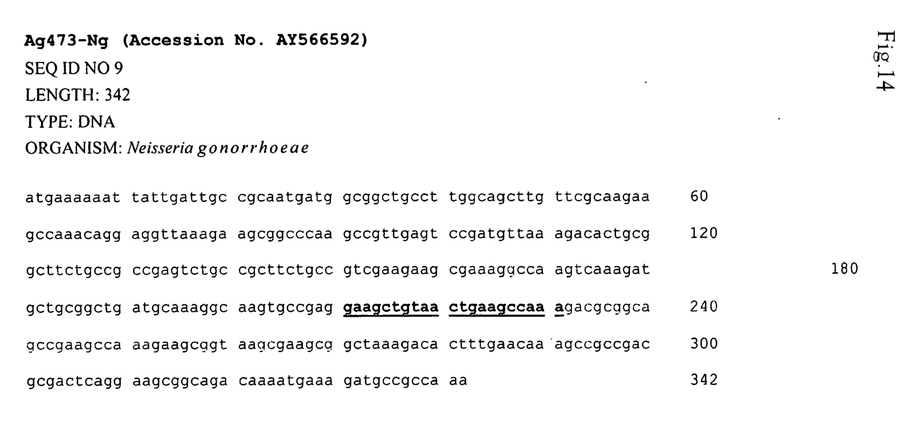

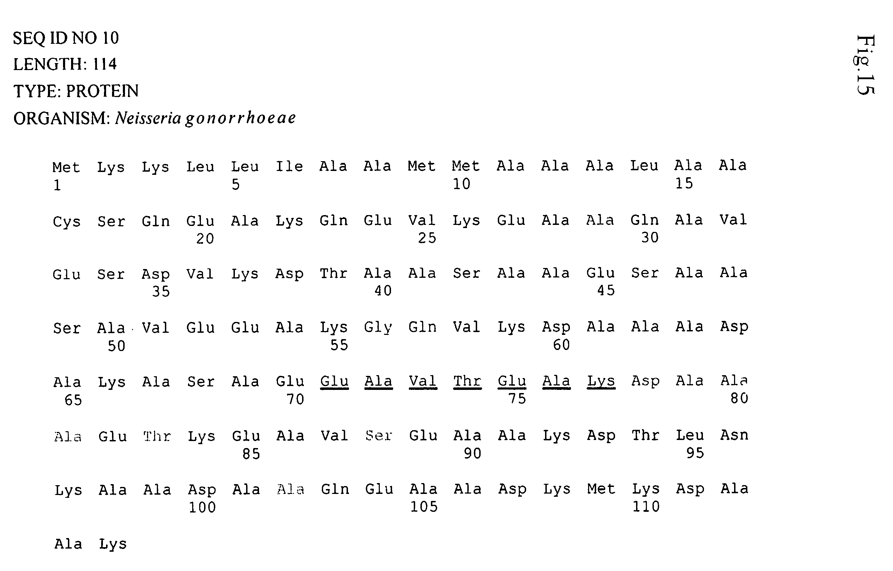

To prove that Ag473 can be used as a vaccine component, recombinant Ag473 is produced in E. coli and used to immunize mice. The ability of the anti-rAg473 to recognize meningococci is demonstrated by whole cell-ELISA and FACS analysis (as shown in FIG.5), wherein Nm22209 is a vaccine strain for preparing the antibody 4-7-3; Nm22209::Ag473(Gm)[Nm22209(Ag473::Gm)] is the mutant with a disrupted Ag473 gene; NM1 and NM2 are the clinical isolates; NM3, NM4 and NM5 are the reference strains of serogroup A, B and C from American Type Culture Collection (ATCC), respectively.

Additionally, In vitro bactericidal activity test as described above shows that the anti-rAg473 is effective in killing NM22208. Accordingly, Ag473 is proved to be able to induce immune response against the whole cells of N. menigitidis and has the potential to be made into a vaccine.

### 5. Expression of Ag473 in N. gonorrhoeae

To examine whether the Ag473 is restricted to N. meningitidis, colony-PCR is performed to detect if the gene is present in other bacteria. Of the strains tested, which include Campylobacter, Haemophilus, Streptococcus, Pertussis, Klebsiella, Staphylococcus, Micrococcus, Enterococcus, Salmonella, Pseudomonas, Shigella, and Neisseria gonorrhoeae, specific product is observed only in N. gonorrhoeae. Sequence analysis shows that the sequence (SEQ ID NO.9) of N. gonorrhoeae PCR product (NG-PCR) reaches 99% identity to that of decoded N. gonorrhoeae genome (as shown in FIG.6). The polynucleotide of NG-PCR reaches 95% of identity to that of SEQ ID NO.1 (Ag473-2) (as shown in FIG.7). The deduced amino acid sequence of NG-PCR reaches > 90% identity to that of Ag473-2 (as shown in FIG.8). Accordingly, it is named Ag473-Ng. Western blot analysis of the total proteins from N. gonorrhoeae with antibody 4-7-3 confirms that the presence of Ag473 protein (FIG.9).

The results indicate that Ag473 may have the potential to act as a vaccine composition against both N. meningitidis and N. gonorrhoeae.

The present invention provides the isolated polypeptide (Ag473), comprising SEQ ID No.2, 4, 6, 8 and 10, recognized by antibody 4-7-3, which can be used to prepare a vaccine or diagnostic or therapeutic reagent.

Besides, the present invention also provides an isolated polynucleotide, comprising SEQ ID No.1, 3, 5, 7 and 9, which can be used to prepare a vaccine or diagnostic or therapeutic reagent. The preferred embodiment herein disclosed is not intended to unnecessarily limit the scope of the invention. Therefore, simple modifications or variations belonging to the equivalent of the scope of the claims and the instructions disclosed herein for a patent are all within the scope of the present invention.

## Claims

1. An isolated lipoprotein comprising an amino acid sequence as shown in SEQ ID NO. 6.

2. A vaccine comprising a lipoprotein according to claim 1.

3. A diagnosis reagent comprising a lipoprotein according to claim 1.

4. A therapy reagent comprising a lipoprotein according to claim 1.

5. An isolated polynucleotide comprising a nucleic acid sequence as shown in SEQ ID NO. 5.

6. A vaccine comprising a polynucleotide according to claim 5.

7. A diagnosis reagent comprising a polynucleotide according to claim 5.

8. A therapy reagent comprising a polynucleotide according to claim 5.

## Patentansprüche

1. Isoliertes Lipoprotein, umfassend eine Aminosäuresequenz wie in SEQ ID-NR. 6 dargestellt.

2. lmpfstoff, umfassend ein Lipoprotein nach Anspruch 1.

3. Diagnose-Reagenz, umfassend ein Lipoprotein nach Anspruch 1.

4. Therapie-Reagenz, umfassend ein Lipoprotein nach Anspruch 1.

5. Isoliertes Polynucleotid, umfassend eine Nukleinsäuresequenz wie in SEQ ID-Nr. 5 dargestellt.

6. Impfstoff, umfassend ein Polynucleotid nach Anspruch 5.

7. Diagnose-Reagenz, umfassend ein Polynucleotid nach Anspruch 5.

8. Therapie-Reagenz, umfassend ein Polynucleotid nach Anspruch 5.

## Revendications

1. Lipoprotéine isolée comprenant une séquence aminoacide comme il est montré dans SEQ ID n° 6.

2. Vaccin comprenant une lipoprotéine selon la revendication 1.

3. Réactif de diagnostic comprenant une lipoprotéine selon la revendication 1.

4. Réactif de thérapie comprenant une lipoprotéine selon la revendication 1.

5. Polynucléotide isolé comprenant une séquence d'acide nucléique comme il est montré dans SEQ ID n° 5.

6. Vaccin comprenant un polynucléotide selon la revendication 5.

7. Réactif de diagnostic comprenant un polynucléotide selon la revendication 5.

8. Réactif de thérapie un polynucléotide selon la revendication 5.
